# EUROPÄISCHE PATENTANMELDUNG

(11) **EP 3 895 640 A1**
(43) Veröffentlichungstag der Anmeldung: **20.10.2021**
(21) Anmeldenummer: 20169721.6
(22) Anmeldetag: 15.04.2020
(51) Int. Cl.: A61B 17/64, A61B 50/30, A61B 17/88

(54) **EINRICHTUNG FÜR DIE AMBULANTE NOTFALLVERSORGUNG**

(71) Anmelder: Kraus, Kilian, 97440 Werneck (DE); Rutencrantz, Andreas, 47506 Neukirchen-Vluyn (DE)
(72) Erfinder: Kraus, Kilian, 97440 Werneck (DE); Rutencrantz, Andreas, 47506 Neukirchen-Vluyn (DE)
(74) Vertreter: Schlögl, Markus

(57) **Zusammenfassung**

Die Erfindung betrifft eine Einrichtung (300) für die ambulante Notfallversorgung, welche zumindest die folgenden Komponenten umfasst:
- einen externen Fixateur (200) mit
- zumindest zwei, beispielsweise vier Pins (220), die zur Verankerung, insbesondere durch Einschrauben, in Knochengewebe ausgebildet sind,
- zumindest ein Stabelement (210) und
- zumindest eine Verbindungsvorrichtung (100), insbesondere zumindest zwei, beispielsweise drei Verbindungsvorrichtungen (100), die dazu ausgebildet sind, zumindest einen Pin (220) mit dem zumindest einen Stabelement (210) mechanisch zu verbinden, und
- eine motorisch angetriebene Befestigungsvorrichtung (301) zum Eintreiben der Pins (220) in das Knochengewebe, wobei die Befestigungsvorrichtung (301) zur einhändigen Bedienung durch einen Benutzer ausgebildet ist.

## Beschreibung

Die Erfindung betrifft eine Einrichtung für die ambulante Notfallversorgung, welche zumindest die folgenden Komponenten umfasst:
- einen externen Fixateur mit
- zumindest zwei, beispielsweise vier Pins, die zur Verankerung, insbesondere durch Einschrauben, in Knochengewebe ausgebildet sind,
- zumindest ein Stabelement und
- zumindest eine Verbindungsvorrichtung, insbesondere zumindest zwei, beispielsweise drei Verbindungsvorrichtungen, die dazu ausgebildet sind, zumindest einen der Pins mit dem zumindest einen Stabelement mechanisch zu verbinden.

Ein externer Fixateur (auch: Fixateur externe) ist eine Vorrichtung zum Stabilisieren und Fixieren einer Fraktur, die insbesondere von Ersthelfern typischerweise bereits am Ort der Verletzung verwendet wird, um zumindest eine temporäre Fixierung der Fraktur zu erreichen und damit beispielsweise die Transportfähigkeit der verletzten Person herzustellen.

Externe Fixateure umfassen in der Regel mehrere miteinander verbindbare bzw. koppelbare Komponenten, um die vorstehend genannte Stabilisierung zu erreichen. Typischerweise umfasst ein externer Fixateur mehrere stangenförmige Pins, die beispielsweise Bohrschraubgewinde aufweisen und dazu ausgebildet sind, in die zu fixierenden Knochenpartien eingetrieben zu werden. Derartige Pins können insbesondere als so genannte Steinmann-Nägel oder Schanzsche Schrauben ausgeführt sein. Die in das Knochengewebe perkutan eingetriebenen Pins werden über eine Art Gestänge außerhalb des Körpers miteinander vergleichsweise starr verbunden, damit sich die Knochenpartien nicht oder nur noch wenig zueinander bewegen können. Zur Verbindung der Pins dienen in der Regel Stabilisierungselemente, die beispielsweise eine längliche, stabförmige oder gekrümmte Gestalt aufweisen und dazu ausgebildet sind, mit Hilfe von Verbindungsvorrichtungen miteinander und/oder mit den Pins verbunden zu werden. Anzahl, Art, Lage und Ausrichtung der zur Stabilisierung der Knochenfraktur eingesetzten Komponenten hängen dabei für gewöhnlich von der Art der Verletzung ab. Bei vergleichsweise einfachen Brüchen kann unter Umständen eine hinreichende Fixierung der Knochenpartien bereits dadurch erreicht werden, dass die in die entsprechenden Knochenpartien eingetriebenen Pins über einen einzelnen Stab bzw. über ein einzelnes Stabilisierungselement, welches die Stelle der Fraktur überbrückt, verbunden werden. Bei komplizierteren Brüchen oder insbesondere bei Brüchen, die die Überbrückung eines Gelenks der verletzten Person erforderlich machen, kann beispielsweise vorgesehen sein, mehrere Stabilisierungselemente miteinander zu koppeln, um eine hinreichende Fixierung zu erreichen.

Externe Fixateure (auch: Fixateur externe, Pin-Fixateur) werden beispielsweise bei geschlossenen oder offenen Brüchen an den Extremitäten, bei denen insbesondere eine Verletzung von Weichgewebe vorliegt, eingesetzt. Eine provisorische Fixierung von derartigen Brüchen mit Hilfe eines externen Fixateurs ist insbesondere in Fällen angezeigt, in denen der oder die Verletzte weitere, lebensbedrohlichere Verletzungen hat, deren Versorgung aus medizinischer Sicht höhere Priorität hat. Typischerweise erfolgt in solchen Fällen eine provisorische Reposition der Fraktur mit anschließender Fixierung in der bereits beschriebenen Art und Weise mit Hilfe eines externen Fixateurs, nachdem der Zustand des oder der Verletzten, beispielsweise im Rahmen von Notfallmaßnahmen, stabilisiert worden ist. Die endgültige Behandlung und Stabilisierung des Bruches, beispielsweise mit Hilfe von Platte, Schraube oder Nagel, im Rahmen der so genannten definitiven Versorgung erfolgt regelmäßig erst dann, wenn der Zustand des Patienten dies erlaubt. Dies kann einige Stunden, meist jedoch einen Tag oder mehrere Tage oder auch eine Woche erfordern.

Die Gefahr einer Infektion am Implantat ist bei höhergradig offenen Frakturen besonders hoch. Bei solchen Brüchen erfolgt die Primärversorgung bevorzugt mit Hilfe von externen Fixateuren, um Infektionen zu vermeiden. Die eigentliche Osteosynthese erfolgt typischerweise in derartig gelagerten Fällen erst nach einigen Tagen unter Zuhilfenahme von Implantaten, wie etwa Platten, Schrauben oder Nägel.

Das Anlegen und Montieren eines externen Fixateurs ist ein relativ komplexer Vorgang. Insbesondere in Notfallsituationen ist es jedoch äußerst vorteilhaft, wenn die Fixierung und Stabilisierung von Frakturen mittels solcher externer Fixateure möglichst zeitnah erfolgt, damit die Transportfähigkeit des Patienten schnellstmöglich hergestellt werden kann.

Aus dem Stand der Technik sind ferner medizinische Einrichtungen mit mehreren Komponenten bekannt, die zur Durchführung von medizinischen Maßnahmen, insbesondere im Rahmen einer Notfallversorgung, dienen. Verbandskästen enthalten beispielsweise verschiedene medizinische Geräte und Verbandsmaterialien, die bei ihrer bestimmungsgemäßen Verwendung zur Behandlung von kleineren und größeren, insbesondere offenen Wunden geeignet sind.

Aus WO 2014/083526 A1 ist eine medizinische Einrichtung zur Durchführung eines chirurgischen Eingriffs bekannt. Die Einrichtung weist nach Art eines Chirurgie-Kits mehrere sterilisierte bzw. sterilisierbare Komponenten auf, die in einer bestimmten Art und Weise auf einer Unterlage organisiert sind. Die Unterlage ist mit gedruckten Illustrationen und/oder Informationen versehen, die die bestimmungsgemäße Verwendung der Komponenten bei der Durchführung von chirurgischen Eingriffen erläutern sollen. Auf diese Weise sollen insbesondere mögliche Fehler, die von ungeübten, nicht hinreichend geschultem medizinischen Personal oder aus Fahrlässigkeit verursacht werden könnten, vermieden werden.

Die Erfindung stellt sich zur Aufgabe, eine Einrichtung für die ambulante Notfallversorgung mit einem externen Fixateur anzugeben, die eine Durchführung einer medizinischen Sofortmaßnahme mit reduziertem Personalaufwand ermöglicht.

Diese Aufgabe wird gelöst durch eine Einrichtung für die ambulante Notfallversorgung mit den Merkmalen des Anspruchs 1.

Vorteilhafte Ausgestaltungen sind Gegenstand der Unteransprüche.

Eine Einrichtung für die ambulante Notfallversorgung umfasst zumindest die folgenden Komponenten:
- einen externen Fixateur mit
- zumindest zwei, beispielsweise vier Pins, die zur Verankerung, insbesondere durch Einschrauben, in Knochengewebe ausgebildet sind,
- zumindest ein Stabelement und
- zumindest eine Verbindungsvorrichtung, insbesondere zumindest zwei, beispielsweise drei Verbindungsvorrichtungen, die dazu ausgebildet sind, zumindest einen der Pins mit dem zumindest einen Stabelement mechanisch zu verbinden. Gemäß der Erfindung ist eine motorisch angetriebene Befestigungsvorrichtung zum Eintreiben der Pins in das Knochengewebe vorgesehen, welche zur einhändigen Bedienung durch einen Benutzer ausgebildet ist.

Der externe Fixateur bildet im montierten Zustand ein zumindest zum größten Teil außerhalb des Körpers eines Verletzten liegendes Gestell, welches zur temporären Fixierung eines Knochenbruches, typischerweise eines offenen Knochenbruches, dient. Neben den Komponenten des externen Fixateurs, wie insbesondere Pins, Stabelementen und Verbindungsvorrichtungen, weist die Einrichtung zudem die motorisch angetriebene Befestigungsvorrichtung zum Eintreiben der Pins in das Knochengewebe und optional weitere medizinische Geräte, wie etwa Wundklemmen, Skalpelle und/oder weiteres medizinisches Zubehör, insbesondere Verbandsmaterialien, für die ambulante Notfallversorgung auf. Die Einrichtung umfasst somit eine Vielzahl von Komponenten und ist insbesondere dazu ausgelegt, im Rahmen des Katastrophenschutzes oder bei militärischen Auseinandersetzungen eingesetzt zu werden insbesondere, wenn davon auszugehen ist, dass eine große Anzahl von Verletzten schnellstmöglich im Rahmen von Notfallmaßnahmen versorgt werden müssen. Derartige Situationen können beispielsweise bei Terroranschlägen oder Naturkatastrophen wie etwa Erdbeben entstehen.

Zur Versorgung eines einzigen Verletzten sind in der Regel zwei Personen notwendig, da insbesondere beim Verankern der Pins in das Knochengewebe im Stand der Technik herkömmliche, insbesondere manuelle, Bohrgeräte zum Einsatz kommen, die von einer Person mit beiden Händen bedient werden muss. Eine weitere Person muss typischerweise die Person während der Notfallmaßnahme fixieren.

Die Befestigungsvorrichtung zum Eintreiben der Pins gemäß der Erfindung ist zum einen motorisch angetrieben und zum anderen zur Betätigung bzw. Bedienung mit nur einer Hand ausgelegt. Im Ergebnis kann daher der externe Fixateur von lediglich einem Ersthelfer angelegt werden. Insbesondere in Rahmen von so genannten Disaster-Recovery Szenarien, in denen eine große Anzahl von Verletzten, beispielswiese mehrere Dutzend oder auch Hunderte von Schwerstverletzten, ambulant versorgt werden müssen, bedeutet dies eine große Erleichterung, da im Durchschnitt pro Zeiteinheit und gegebener Anzahl von Ersthelfern doppelt so viele Verletzte behandelt werden können.

Die Einrichtung zur ambulanten Notfallversorgung enthält typischerweise alle medizinischen Komponenten, die notwendig sind, um Knochenfragmente mit Hilfe des externen Fixateurs zu stabilisieren. In Ausgestaltungen kann die Einrichtung zur ambulanten Notfallversorgung weitere medizinische Komponenten aufweisen, so dass mittels der Einrichtung zur ambulanten Notfallversorgung auch andere medizinische Maßnahmen durchgeführt werden können.

In Ausgestaltungen ist die Befestigungsvorrichtung als elektrischer Schrauber mit T-förmigen Griffstück ausgebildet. Die Befestigungsvorrichtung ist beispielsweise ergonomisch zur Betätigung und Bedienung mit einer Hand ausgebildet.

In Ausgestaltungen weist die Befestigungsvorrichtung eine Aufnahme zur kraftschlüssigen Befestigung eines der Pins auf. Die Aufnahme der Befestigungsvorrichtung ist beispielsweise nach Art eines Spannfutterals ausgebildet.

In Ausgestaltungen weist die Befestigungseinrichtung einen Energiespeicher, insbesondere eine Einwegbatterie oder eine wiederaufladbare Batterie, auf. Die Einrichtung ist Ausgestaltungen zur einmaligen oder mehrmaligen Verwendung vorgesehen.

In Ausgestaltungen umfasst die Einrichtung zumindest zwei Stabelemente (auch:
Stäbe, Stangen, Stangenelemente), die optional unterschiedliche axiale Längen aufweisen. Die zumindest eine Verbindungsvorrichtung ist dazu ausgebildet, die zumindest zwei Stabelemente miteinander mechanisch zu verbinden. Mit Hilfe der Verbindungsvorrichtung könne somit nicht nur die Pins an den Stabelementen befestigt werden, sondern auch die Stangenelemente untereinander. Eine derartige Ausgestaltung ist insbesondere bei der Behandlung von komplizierteren Brüchen, die beispielsweise eine gelenküberbrückende Konfiguration erfordern, wünschenswert.

In Ausgestaltungen weisen zumindest zwei der Pins unterschiedliche axiale Längen und/oder Durchmesser auf. Pins mit unterschiedlichen Durchmessern und/oder axialen Längen können beispielsweise für unterschiedlich medizinische Anwendungszwecke ausgelegt sein. Die verschiedenen Pins können insbesondere zur Verankerung im Schienbein (lat.: Tibia), im Oberschenkelknochen (lat.: Femur), in der Elle (lat.: Ulna), in der Speiche (lat.: Radius), im Oberarmknochen (lat.: Humerus), im Becken (lat.: Pelvis) oder in Mittelhandknochen (lat.: Ossa metacarpi) ausgebildet sein.

In Ausgestaltungen sind zumindest einige der Komponenten der Einrichtung in einer Kunststoffhülle eingeschweißt. Auf diese Weise kann einer Kontaminierung bzw. Verunreinigung der Komponenten der Einrichtung entgegengewirkt werden.

In Ausgestaltungen sind die in der Kunststoffhülle eingeschweißten Komponenten sterilisiert oder sterilisierbar. Vorzugsweise sind zumindest solche Komponenten, die bei der Verwendung der Einrichtung in direktem Kontakt mit menschlichem Gewebe kommen, wie etwa insbesondere die Pins und/oder etwaig vorhandene Skalpells, Klemmen oder dergleichen, steril verpackt.

In Ausgestaltungen weist die Einrichtung eine textile Unterlage zur Bereitstellung einer Eingriffsumgebung auf. Die textile Unterlage kann insbesondere auf dem Boden ausgebreitet werden, um behelfsmäßig eine zumindest vergleichsweise saubere Eingriffsumgebung zu schaffen, auf der die weiteren medizinischen Notfallmaßnahmen, insbesondere zum Anlegen des externen Fixateurs, durchgeführt werden können. Die textile Unterlage weist hierzu geeignete Dimensionen auf. Im verpackten Zustand können die übrigen Komponenten der Einrichtung, die beispielsweise an der bereits beschriebenen Kunststoffhülle befestigt oder in bereits beschriebenen Kunststoffhülle eingeschweißt sind, in Ausgestaltungen eingewickelt sein.

In Ausgestaltungen ist die zumindest eine Verbindungsvorrichtung dazu ausgebildet, die zumindest zwei Pins mit zumindest einem der Stabelemente mechanisch zu verbinden. Da die Verbindungsvorrichtung dazu ausgebildet ist, mehrere Pins am Stabelement zu befestigen, kann die Anzahl der zur Montage des externen Fixateurs notwendig Verbindungsvorrichtung reduziert werden. Dies reduziert in vorteilhafter Weise die Herstellungskosten der Einrichtung und deren Gesamtgewicht.

In Ausgestaltungen ist die zumindest eine Verbindungsvorrichtung dazu ausgebildet, die zumindest zwei Pins in paralleler Ausrichtung mit zumindest einem der Stabelemente mechanisch zu verbinden. Die an der Verbindungseinrichtung befestigten Pins können beispielsweise dazu dienen, ein einzelnes Knochenfragment zu fixieren.

Die mechanische Verbindung der Pins mit den Stabelementen bzw. der Stabelemente untereinander erfolgt typischerweise zumindest unter Vermittlung eines Kraftschlusses. In Ausgestaltungen erfolgt die mechanische Verbindung alternativ oder zusätzlich unter Vermittlung eines Formschlusses, der durch einander komplementär ausgebildete Oberflächenstrukturierungen vermittelt wird.

Die Verbindungsvorrichtung zur Verbindung von Pins und/oder Stabelementen des externen Fixateurs umfasst in Ausgestaltungen eine erste Klemmenanordnung zur Befestigung zumindest eines Pins und/oder Stabelements und eine zweite Klemmenanordnung zur Befestigung zumindest eines Pins und/oder Stabelements. Die ersten und zweiten Klemmenanordnungen sind beispielsweise über ein Drehgelenk um eine Drehachse zueinander drehbar und über ein Schwenkgelenk um eine senkrecht zur Drehachse verlaufende Schwenkachse zueinander schwenkbar gelagert. In bevorzugten Ausgestaltungen ist ein zugbelastbares Zugmittel durch die erste und zweite Klemmenanordnung, das Drehgelenk und das Schwenkgelenk hindurchgeführt. Das zugbelastbare Zugmittel ist dazu ausgebildet, durch Vermittlung einer Zugkraft (auch: Zugspannung, Zugbelastung) in axialer Richtung längs des Zugmittels die erste und zweite Klemmenanordnung zu fixieren und bezüglich der Drehachse und der Schwenkachse in unterschiedlichen Winkelstellung zu arretieren. Das Zugmittel weist insbesondere eine in axialer Richtung ausgedehnte, längliche Gestalt auf und ist beispielsweise einstückig oder zumindest starr ausgebildet. Die axiale Richtung ist insbesondere von der Längsausdehnung des Zugmittels vorgegeben. Da das einteilige oder starre Zugmittel sowohl durch die erste und zweite Klemmenanordnung als auch durch das Drehgelenk und das Schwenkgelenk hindurchgeführt ist, können alle Freiheitsgrade der Verbindungsvorrichtung durch Vermittlung einer Zugkraft in axialer Richtung längs des Zugmittels fixiert werden. Diese Freiheitsgrade betreffen zum einen die Rotationen der Klemmenanordnungen zueinander um die Schwenkachse und um Drehachse. Zum anderen werden auch die Stabelemente und/oder Pins in den Klemmenanordnungen durch die Vermittlung der Zugkraft in axialer Richtung längs des Zugmittels kraftschlüssig befestigt. Hierzu weist die erste Klemmenanordnung und die zweite Klemmenanordnung beispielsweise gegeneinander verspannbare erste und zweite Klemmbacken auf. Zur vollständigen Fixierung und Arretierung der Verbindungsvorrichtung muss somit lediglich eine längs des Zugmittels wirkende Zugspannung vermittelt werden, was beispielsweise durch Anziehen eines einzigen Stellmittels, wie etwa einer Stellschraube oder einer Schraubenmutter, erfolgen kann. Der Bedienaufwand zum Anbringen eines derartige Verbindungsvorrichtungen aufweisenden externen Fixateurs ist daher reduziert. Zudem ist die Funktionsweise des externen Fixateurs intuitiv besonders leicht zu erfassen, so dass dieser auch von einer medizinisch ungeschulten oder zumindest vergleichsweise unerfahrenen Person insbesondere im Rahmen einer notfallmedizinischen Maßnahme angelegt werden kann.

Das Zugmittel ist in Ausgestaltungen beispielsweise als Schraube ausgeführt. Die axiale Richtung entspricht in derartigen Ausführungen der Richtung, in der die Längsmittelachse der Schraube orientiert ist. Im Allgemeinen weicht die axiale Richtung von der Richtung der Drehachse ab, da die Orientierung der Drehachse von der Winkelstellung der Klemmenanordnungen um die Schwenkachse abhängt. In einer Grundstellung, in der die Klemmenanordnungen zueinander nicht bezüglich der Schwenkachse verstellt sind, verläuft die Drehachse koaxial zur axialen Richtung, die im Wesentlichen von der Längsausdehnung des Zugmittels definiert ist.

In Ausgestaltungen umfasst das Drehgelenk zumindest zwei zueinander um die Drehachse drehbar gelagerte und aneinander angrenzende Drehgelenkkomponenten, die mittels des Zugmittels relativ zueinander in unterschiedlichen Winkelstellung arretierbar sind. Zwischen den Drehgelenkkomponenten ist ein erstes Federmittel angeordnet, welches dazu ausgebildet ist, die Drehgelenkkomponenten in axialer Richtung voneinander weg zu spannen. Das Zugmittel ist beispielsweise zentral durch die das Drehgelenk bildenden Drehgelenkkomponenten geführt. Das erste Federmittel bewirkt eine in axialer Richtung wirkende Federkraft, die dazu ausgelegt ist, die Drehgelenkkomponenten auseinander zu treiben, so dass diese im unbelasteten Zustand, in dem das Zugmittel nicht oder nur geringfügig unter Zugbelastung steht, gegeneinander in die gewünschte Winkelstellung, insbesondere händisch, bewegt werden können. Durch Vermittlung der Zugkraft in axialer Richtung längs des Zugmittels können die Drehgelenkkomponenten anschließend in der gewünschten Winkelstellung arretiert werden, d. h. die zur Arretierung der Verbindungsvorrichtung wirkende Zugbelastung ist entgegen der Federkraft gerichtet, die vom ersten Federmittel vermittelt wird.

In Ausgestaltungen ist das erste Federmittel beispielsweise in der Art einer Wellenfeder ausgeführt. Das erste Federmittel ist vorzugsweise dazu ausgelegt, Querkräfte aufzunehmen, die beispielsweise in Richtungen auf das Drehgelenk bzw. auf die Drehgelenkkomponenten einwirken, die von der Richtung, in der die Drehachse orientiert ist, abweichen. In Ausgestaltungen ist hierzu das erste Federmittel als biegebelastbare oder biegebelastete Zylinderbalkenfeder ausgeführt.

Die aneinander angrenzenden Drehgelenkkomponenten implementieren zusammen mit dem Zugmittel, das beispielsweise zentral durch die Drehgelenkkomponenten geführt ist, in Ausgestaltungen die Funktionalität des Drehgelenks, also der Rotation der ersten und zweiten Klemmenanordnungen zueinander und um die Drehachse. In Ausgestaltungen, in denen das Zugmittel zentral durch die Drehgelenckomponenten geführt ist, sind diese zueinander und um das Zugmittel vorzugsweise über einen Winkelbereich von 360° drehbar.

Die Drehgelenkkomponenten sind in Ausgestaltungen beispielsweise im Wesentlichen scheibenförmig ausgeführt und einander stirnseitig gegenüberliegend angeordnet.

In Ausgestaltungen ist die erste Klemmenanordnung mit einer der Drehgelenkkomponenten und die zweite Klemmenanordnung mit der jeweilig anderen Drehgelenckomponente zumindest bezüglich einer Rotation um die Drehachse drehfest verbunden. In Ausgestaltungen ist zumindest eine der Drehgelenkkomponenten als integrales Bauteil der ersten oder zweiten Klemmenanordnung realisiert. Beispielsweise bildet zumindest eine der Drehgelenkkomponenten eine (erste oder zweite) Klemmbacke der ersten oder zweiten Klemmenanordnung. Mit anderen Worten ist in Ausgestaltungen beispielsweise vorgesehen, zumindest eine der Drehgelenkkomponenten einstückig mit einer (ersten oder zweiten) Klemmbacke der ersten und/oder zweiten Klemmenanordnung auszubilden. Auf diese Weise kann die Anzahl der zur Ausbildung der Verbindungsvorrichtung notwendigen Komponenten reduziert werden und so insbesondere Gewicht eingespart werden. Dies ist insbesondere vorteilhaft bei einem Einsatz des hier vorgestellten externen Fixateurs im Bereich des Katastrophenschutzes und/oder des Militärs, da dort die Notwendigkeit bestehen kann, den externen Fixateur als Teil medizinischer Notfallausrüstung im Gelände, insbesondere über längere Strecken, mitzuführen.

In Ausgestaltungen weisen die Drehgelenkkomponenten einander gegenüberliegend angeordnete und zueinander komplementär ausgebildete erste Oberflächenstrukturierungen auf, die dazu ausgebildet sind, in unterschiedlichen Winkelstellungen formschlüssig ineinander einzugreifen. Entsprechend erfolgt die Arretierung des rotatorischen Freiheitsgrades der Verbindungsvorrichtung bezüglich der Verstellung der Klemmenanordnungen um die Drehachse unter zusätzlicher Zuhilfenahme eines Formschlusses. Auf diese Weise kann die zur Arretierung dieses Freiheitsgrades notwendige Kraft im Vergleich zu Ausführungen, die ausschließlich auf einer kraft- und/oder reibschlüssigen Arretierung beruhen, deutlich vermindert werden und die mechanische Stabilität des montierten externen Fixateurs verbessert werden. Insbesondere kann so erreicht werden, dass die zur hinreichenden Fixierung der mittels der Verbindungsvorrichtung verbundenen Komponenten notwendige Zugbelastung durch eine Person werkzeuglos bzw. händisch, beispielsweise durch Anziehen eines einzigen, mit dem Zugmittel in einer Wirkverbindung stehenden Stellmittels vermittelt werden kann. Dieses Stellmittel ist beispielsweise ein Handrad, ein Sterngriff, eine Schraube oder eine Schraubenmutter, welches zur Vermittlung der Zugbelastung mit dem Zugmittel in einer Wirkverbindung steht.

Die erste Oberflächenstrukturierung weist in Ausgestaltungen beispielsweise eine zumindest bereichsweise gezahnte oder geriffelte Struktur auf. In Ausgestaltungen sind die ersten Oberflächenstrukturierungen beispielsweise als in Richtung der Drehachse axial vorstehende, umlaufende Zahnkränze ausgeführt, die einander gegenüberliegend angeordnet sind und als Stirnverzahnung dazu ausgebildet sind, ineinander formschlüssig einzugreifen.

In Ausgestaltungen umfasst das Schwenkgelenk zumindest zwei zueinander um die Schwenkachse schwenkbar gelagerte und aneinander angrenzende Schwenkgelenckomponenten, die mittels des Zugmittels relativ zueinander in unterschiedlichen Winkelstellung arretierbar sind. Zwischen den Schwenkgelenkkomponenten ist ein zweites Federmittel angeordnet, welches dazu ausgebildet ist, die Schwenkgelenckomponenten in axialer Richtung voneinander weg zu spannen. Das Zugmittel ist beispielsweise zentral durch die das Schwenkgelenk bildenden Schwenkgelenkkomponenten geführt. Das zweite Federmittel bewirkt eine in axialer Richtung wirkende Federkraft, die dazu ausgelegt ist, die Schwenkgelenkkomponenten auseinander zu treiben, so dass diese im unbelasteten Zustand, in dem das Zugmittel nicht oder nur geringfügig unter Zugbelastung steht, gegeneinander in die gewünschte Winkelstellung, insbesondere werkzeuglos bzw. händisch, bewegt werden können. Durch Vermittlung der Zugkraft in axialer Richtung längs des Zugmittels können die Schwenkgelenkkomponenten anschließend in der gewünschten Winkelstellung arretiert werden, d. h. die zur Arretierung der Verbindungsvorrichtung wirkende Zugbelastung ist entgegen der Federkraft gerichtet, die vom zweiten Federmittel vermittelt wird.

In Ausgestaltungen ist das zweite Federmittel beispielsweise als Blattfeder ausgeführt. Die Blattfeder weist in Ausgestaltungen eine mittig angeordnete Öffnung auf, durch die das Zugmittel hindurchgeführt ist.

Die aneinander angrenzenden Schwenkgelenkkomponenten implementieren zusammen mit dem Zugmittel, das beispielsweise zentral durch die Schwenkgelenkkomponenten geführt ist, im Wesentlichen die Funktionalität des Schwenkgelenks, also der Rotation der ersten und zweiten Klemmenanordnungen zueinander und um die Schwenkachse. In Ausgestaltungen sind die Schwenkgelenkkomponenten relativ zueinander über einen eingeschränkten Winkelbereich von weniger als 180°, insbesondere über einen Winkelbereich von weniger als 90° schwenkbar. In Ausgestaltungen sind die Schwenkgelenkkomponenten bzw. die erste Klemmenanordnung relativ zur zweiten Klemmenanordnung über einen Winkelbereich von zumindest 40°, beispielsweise in einem Winkelbereich von etwa +/- 20° gegenüber einer Grundstellung, schwenkbar.

In Ausgestaltungen weist zumindest eine der Schwenkgelenkkomponenten ein zentrales Langloch auf, durch welches das Zugmittel geführt ist. Das Langloch bildet eine Art Kulisse, entlang der das zentral geführte Zugmittel bewegbar ist, so dass eine Verstellung der beiden Klemmenanordnungen zueinander um die Schwenkachse über einen entsprechend großen Winkelbereich ermöglicht ist.

Die Schwenkgelenkkomponenten sind beispielsweise im Wesentlichen scheibenförmig ausgeführt und einander gegenüberliegend angeordnet. Die gegenüberliegend angeordneten Seiten der Schwenkgelenkkomponenten weisen beispielsweise zumindest abschnittsweise gekrümmte Abschnitte auf, die die Bewegung der beiden Schwenkgelenkkomponenten zueinander im Wesentlichen auf eine Schwenkbewegung senkrecht zur Drehachse einschränken. Die einander zugewandten Seiten der Schwenkgelenkkomponenten sind beispielsweise so geformt, dass die Schwenkgelenkkomponenten zueinander nicht um die Drehachse verstellt werden können. Mit anderen Worten bilden die Schwenkgelenkkomponenten mit dem Zugmittel in derartigen Ausgestaltungen ein Schwenkgelenk, welches die erste Klemmenanordnung mit der zweiten Klemmenanordnung hinsichtlich Rotationen um die Drehachse drehfest verbindet.

In Ausgestaltungen ist die erste Klemmenanordnung mit einer der Schwenkgelenckomponenten und die zweite Klemmenanordnung mit der anderen Schwenkgelenckomponente zumindest bezüglich einer Rotation um die Schwenkachse drehfest verbunden. In Ausgestaltungen ist zumindest eine der Schwenkgelenkkomponenten als integrales Bauteil der ersten oder zweiten Klemmenanordnung realisiert. Beispielsweise bildet zumindest eine der Schwenkgelenkkomponenten eine (erste oder zweite) Klemmbacke der ersten oder der zweiten Klemmenanordnung. Mit anderen Worten ist in Ausgestaltungen beispielsweise vorgesehen, zumindest eine der Schwenkgelenkkomponenten einstückig mit einer (ersten oder zweiten) Klemmbacke der ersten oder zweiten Klemmenanordnung auszuführen. Auch auf diese Weise kann die Anzahl der zur Ausbildung der Verbindungsvorrichtung notwendigen Komponenten reduziert werden und so insbesondere Gewicht und/oder Materialkosten eingespart werden.

In Ausgestaltungen ist vorgesehen, dass die Funktion einer der Schwenkgelenkkomponenten und die Funktion einer der Drehgelenkkomponenten durch ein einziges Bauteil realisiert ist. Dieses Bauteil fungiert entsprechend gleichermaßen als Dreh- und als Schwenkgelenkkomponente, um die Anzahl der zur Realisierung der Verbindungsvorrichtung notwendigen Komponenten weiter zu reduzieren. Das Drehgelenk und das Schwenkgelenk ist in Ausgestaltungen beispielsweise durch lediglich drei, insbesondere im Wesentlichen scheibenförmig ausgeführte, Bauteile gebildet, die in axialer Richtung aufeinanderfolgend aneinander angrenzen und eine Verbindung zwischen der ersten und der zweiten Klemmenanordnung derart bereitstellen, dass diese sowohl bezüglich der Schwenk- als auch der Drehachse zueinander in unterschiedlichen Winkelstellungen positioniert werden können. In Ausgestaltungen, in denen (erste oder zweite) Klemmbacken der ersten und/oder der zweiten Klemmenanordnungen zusätzlich als Drehgelenk- oder Schwenkgelenkkomponenten fungieren, ist die Anzahl der Bauteile noch weiter reduziert. Auf diese Weise wird der zur Realisierung der Verbindungsvorrichtung erforderliche Bauraum in axialer Richtung verkleinert und zusätzliches Gewicht eingespart.

In Ausgestaltungen weisen die Schwenkgelenkkomponenten einander gegenüberliegend angeordnete und zueinander komplementär ausgebildete zweite Oberflächenstrukturierungen auf, die dazu ausgebildet sind, in unterschiedlichen Winkelstellungen formschlüssig ineinander einzugreifen. Entsprechend erfolgt die Arretierung des rotatorischen Freiheitsgrades der Verbindungsvorrichtung bezüglich der Verstellung der ersten und zweiten Klemmenanordnungen um die Schwenkachse unter zusätzlicher Zuhilfenahme eines Formschlusses. Auf diese Weise kann die zur Arretierung dieses Freiheitsgrades notwendige Kraft im Vergleich zu Ausführungen, die ausschließlich auf einer kraft- und/oder reibschlüssigen Arretierung beruhen, deutlich vermindert werden und die mechanische Stabilität des montierten externen Fixateurs verbessert werden. Die zur hinreichenden Fixierung der mittels der Verbindungsvorrichtung verbundenen Komponenten des externen Fixateurs notwendige Zugbelastung kann insbesondere durch eine Person werkzeuglos bzw. händisch, beispielsweise durch Anziehen eines einzigen mit dem Zugmittel in einer Wirkverbindung stehenden Stellmittels, vermittelt werden. Dieses Stellmittel ist beispielsweise ein Sterngriff, ein Handrad, eine Schraube oder eine Schraubenmutter, welches zur Vermittlung der Zugbelastung mit dem Zugmittel in einer Wirkverbindung steht.

Die zweiten Oberflächenstrukturierungen weisen in Ausgestaltungen beispielsweise zumindest bereichsweise gezahnte oder geriffelte Strukturen auf. In Ausgestaltungen umfassen die zweiten Oberflächenstrukturierungen beispielsweise mehrere parallel zur Schwenkachse verlaufende Rillen, Riefen und/oder gratähnlich ausgebildete Rippen auf. Die gratähnlichen Rippen sind in Ausgestaltungen beispielsweise komplementär zu den Rillen bzw. Riefen ausgebildet. Zur Bereitstellung des Formschlusses werden die gratähnlichen Rippen der einen Schwenkgelenkkomponente den Rillen bzw. Riefen der anderen Schwenkgelenkkomponente gegenüberliegend angeordnet, so dass diese nach Art einer Stirnverzahnung ineinander eingreifen können.

In Ausgestaltungen weist bzw. weisen die erste Klemmenanordnung und/oder die zweite Klemmenanordnung zwei gegenüberliegende, gegeneinander durch Vermittlung der in axialer Richtung wirkenden Zugkraft verspannbare, erste und zweite Klemmbacken auf, die jeweils gegenüberliegend angeordnete erste Aufnahmenuten zur Aufnahme eines Stabelements umfassen. Die Stabelemente (auch: Stäbe, Stangen) des externen Fixateurs können in die Aufnahmenuten eingelegt werden und mit Hilfe der bereits beschriebenen Verbindungsvorrichtung in unterschiedlichen räumlichen Konfigurationen verbunden und durch Ausüben der axialen Zugbelastung kraft- bzw. reibschlüssig arretiert werden.

In Ausgestaltungen sind die ersten Aufnahmenuten zumindest abschnittsweise mit dritten Oberflächenstrukturierungen versehen, die dazu ausgebildet sind, in komplementär ausgebildete Oberflächenstrukturierungen eines Stabelements formschlüssig einzugreifen. In Ausgestaltungen umfasst die dritte Oberflächenstrukturierung beispielsweise mehrere parallel zueinander verlaufende Rillen, Riefen und/oder gratähnlich ausgebildete Rippen. Die Fixierung der Stabelemente erfolgt in derartigen Ausführung somit unter zusätzlicher Zuhilfenahme eines Formschlusses.

In Ausgestaltungen weist bzw. weisen die erste Klemmenanordnung und/oder die zweite Klemmenanordnung zwei gegenüberliegende, gegeneinander durch Vermittlung der in axialer Richtung wirkenden Zugkraft verspannbare, erste und zweite Klemmbacken auf, die jeweils gegenüberliegend angeordnete zweite Aufnahmenuten zur Aufnahme eines Pins umfassen. Der Pin kann in die Aufnahmenuten der gegenüberliegenden ersten und zweiten Klemmbacken eingelegt werden und durch Ausüben der axialen Zugbelastung kraft- bzw. reibschlüssig arretiert werden. Der Pin ist dazu ausgebildet, im Knochengewebe einer verletzten Person bzw. eines Patienten eingetrieben zu werden und weist dazu beispielsweise ein Bohrgewinde auf. Typischerweise wird der Pin mit der Verbindungsvorrichtung fest verbunden, nachdem dieser im Knochengewebe verankert wurde. In Ausgestaltungen sind die Pins als Gewindestangen, insbesondere aus Metall, ausgeführt. In Ausgestaltungen weisen die Stabelemente und die Pins unterschiedliche Durchmesser auf, so dass die ersten und zweiten Aufnahmenuten entsprechend unterschiedlich dimensioniert sind.

In Ausgestaltungen weisen die ersten und zweiten Klemmbacken zwei zueinander parallel verlaufende zweite Aufnahmenuten zur Aufnahme jeweils eines Pins auf. Zum Fixieren eines Bruches mit Hilfe des externen Fixateurs werden typischerweise mehrere Pins, zumeist zwei Pins, in jedes einzelne Knochenfragment eingetrieben, die entsprechend relativ nahe beieinander angeordnet werden. Mit Hilfe einer einzigen Verbindungsvorrichtung können bereits zwei Pins mit beispielsweise einem Stabelement des externen Fixateurs verbunden werden, so dass die Anzahl der zur Stabilisierung der Fraktur notwendigen Komponenten reduziert ist. Zudem ist der Montageaufwand reduziert, da die beiden in die zweiten Aufnahmenuten eingelegten Pins durch Vermittlung der Zugkraft in axialer Richtung kraft- bzw. reibschlüssig fixiert werden, wobei auch alle übrigen Freiheitsgrade der Verbindungsvorrichtung, insbesondere die Ausrichtung der ersten und zweiten Klemmenanordnungen bezüglich der Dreh- und der Schwenkachse und ein etwaig in die ersten Aufnahmenuten eingelegtes Stabelement arretiert werden.

In Ausgestaltungen sind die ersten und zweiten Klemmbacken mit ineinander eingreifenden Strukturen versehen, die dazu ausgebildet sind, eine Rotation der ersten und zweiten Klemmbacken zueinander zu blockieren. Beispielsweise weist eine der ersten oder zweiten Klemmbacken zumindest einen stirnseitig hervorstehenden Stift auf, der in eine sacklochartige Vertiefung, die in die jeweils andere der ersten oder zweiten Klemmbacken eingebracht ist, eingreift. Durch diese Maßnahme wird sichergestellt, dass die in den ersten und zweiten Klemmbacken eingebrachten ersten und/oder zweiten Aufnahmenuten stets parallel verlaufen.

In Ausgestaltungen ist das Zugmittel endseitig in einem Gelenk, insbesondere Kugelgelenk, der Verbindungsvorrichtung beweglich geführt, beispielsweise derart, dass das Zugmittel um zwei zueinander senkrecht verlaufende Achsen schwenkbar ist. Das insbesondere starr ausgebildete Zugmittel ist somit beweglich gelagert, so dass die erste und die zweite Klemmenanordnung zueinander insbesondere bezüglich der Schwenk- und der Drehachse verstellt werden kann. Beispielsweise ist das Gelenk in einem zentralen Bereich der Klemmbacke der ersten oder zweiten Klemmenanordnung gebildet, die bezüglich der axialen Richtung endseitig angeordnet ist.

In Ausgestaltungen ist das Zugmittel als Schraube mit einem zumindest bereichsweise kugelförmig ausgebildeten Kopfende ausgeführt, wobei das Kopfende in einer Gelenkpfanne des Gelenks geführt ist. Die Gelenkpfanne ist in Ausgestaltungen beispielsweise mittig bzw. in einem zentralen Bereich einer endseitig angeordneten Komponente der Verbindungvorrichtung angeordnet. Die Gelenkpfanne mit dem darin geführten Kopfende bildet eine Art Kugelgelenk, so dass die Schraube bezüglich zweier zueinander senkrecht und durch das Gelenk verlaufende Achsen im Wesentlichen frei verschwenkbar ist.

In Ausgestaltungen, in denen das Zugmittel als Schraube ausgeführt ist, ist die axiale Richtung von der Ausrichtung der Mittellängsachse der Schraube vorgegeben. Die Schraube weist insbesondere ein Außengewinde auf, welches als Teil- oder Vollgewinde ausgeführt ist. Ein Stellmittel, insbesondere ein Handrad, ein Sterngriff, eine Schraube oder eine Schraubenmutter, ist zur Vermittlung der Zugbelastung auf das Außengewinde aufschraubbar bzw. kann zur Vermittlung der Zugbelastung auf das Außengewinde aufgeschraubt werden.

In Ausgestaltungen ist in das Kopfende des Zugmittels zumindest eine Nut eingebracht, in die ein der Gelenkpfanne hervorstehender Vorsprung oder eine der Gelenkpfanne hervorstehende Nase derart eingreift, dass eine Rotation der Schraube um die axiale Richtung zumindest eingeschränkt ist. Mit dieser Maßnahme wird eine Rotation der Schraube um ihre Mittellängsachse bzw. um die axiale Richtung zumindest weitgehend unterbunden, so dass das Stellmittel, insbesondere das Handrad, der Sterngriff, die Schraube oder die Schraubenmutter, zur Vermittlung der Zugkraft einfach auf das Gewinde der Schraube aufgeschraubt werden kann. Mit anderen Worten bildet der in die Nut eingreifende Vorsprung eine Art Drehmomentstütze für das um die Mittellängsachse wirkende Anzugsmoment, welches zur Vermittlung der Zugkraft in axialer Richtung ausgeübt wird. Die Wechselwirkung zwischen Vorsprung und Nut schränkt dabei die vorstehend beschriebene Beweglichkeit der Schraube hinsichtlich einer Verschwenkung um die durch das Gelenk verlaufenden Achsen nicht oder nur unwesentlich ein.

Der externe Fixateur weist in Ausgestaltungen zumindest eine Verbindungsvorrichtung auf, die wie vorstehend bereits beschrieben ausgebildet ist. Der externe Fixateur weist grundsätzlich einen modularen Aufbau mit mehreren miteinander verbindbaren Komponenten auf. Anzahl und Ausführung der Komponenten, insbesondere hinsichtlich ihrer Dimensionierung, kann variieren. Je nach Art und Schwere der Fraktur kann aus medizinischer Sicht insbesondere eine unterschiedliche Anzahl von Stabelementen, Pins und/oder Verbindungsvorrichtungen vorgesehen sein, um eine hinreichende Stabilisierung der Fraktur zu bewirken. Zudem können insbesondere Stabelemente mit unterschiedlichen axialen Längen und/oder Pins mit unterschiedlichen Durchmessern und/oder axialen Längen vorgesehen sein, die beispielsweise zur Verankerung im Schienbein (lat.: Tibia), im Oberschenkelknochen (lat.: Femur), in der Elle (lat.: Ulna), in der Speiche (lat.: Radius), im Oberarmknochen (lat.: Humerus), im Becken (lat.: Pelvis) oder in Mittelhandknochen (lat.: Ossa metacarpi) ausgebildet sind. Es versteht sich, dass diese Aufzählung nicht abschließend ist.

Der externe Fixateur umfasst
- zumindest zwei Pins, die zur Verankerung, insbesondere durch Einschrauben, in Knochengewebe ausgebildet sind,
- zumindest ein Stabelement und
- zumindest eine der vorstehend bereits beschriebenen Verbindungsvorrichtungen.
   Der externe Fixateur umfasst in Ausgestaltungen
- zumindest vier Pins, vorzugsweise sechs oder acht Pins, die zur Verankerung, insbesondere durch Einschrauben, in Knochengewebe ausgebildet sind,
- zwei Stabelemente, insbesondere unterschiedlicher axialer Längen, und
- zumindest zwei, beispielsweise drei oder vier der vorstehend bereits beschriebenen Verbindungsvorrichtungen.

Es versteht sich, dass der externe Fixateur eine beliebige Anzahl von Verbindungsvorrichtungen, Pins und/oder Stabelementen aufweisen kann. Der externe Fixateur weist in vorteilhaften Ausgestaltungen jedoch keine Komponenten oder Bauteile auf, die strukturell von den genannten Verbindungsvorrichtungen, Pins und/oder Stabelementen verschieden sind. Durch das Vorsehen von möglichst wenigen unterschiedlich ausgebildeten Komponenten ist die Handhabung und Montage des externen Fixateurs stark vereinfacht, da mögliche Fehlerquellen, die insbesondere aus einer falsche Verwendung eines bestimmten Bauteils bei der Montage des externen Fixateurs resultieren, vermieden werden können.

Der externe Fixateur ist in Ausgestaltungen beispielsweise zur mehrfachen Verwendung vorgesehen bzw. ausgeführt. In anderen Ausgestaltungen ist der externe Fixateur zur einmaligen Verwendung vorgesehen bzw. ausgeführt. In diesem Fall ist eine mögliche Abnutzung von Oberflächenstrukturierungen, die zur Bereitstellung eines Formschlusses zwischen unterschiedlichen Komponenten des externen Fixateurs oder zwischen unterschiedlichen Bauteilen der Verbindungsvorrichtung eher unkritisch.

Bei einem Verfahren zur Durchführung einer medizinischen Maßnahme der ambulanten Notfallversorgung werden zumindest einige der Pins in Knochenfragmente eines Verletzten bzw. eines Patienten mit Hilfe der zur einhändigen Bedienung durch einen Benutzer ausgebildeten Befestigungsvorrichtung eingetrieben. Vorzugsweise erfolgt dies unter Verwendung der bereits beschriebenen, motorisch angetriebenen Befestigungseinrichtung, die in Ausgestaltungen zur besseren Handhabung mit einem T-förmigen Griffstück ausgebildet ist.

Gegebenenfalls wird vor dem Eintreiben der Pins ein Zugang zu den Knochenfragmenten geschaffen, beispielsweise in Form eines Kanals, welcher insbesondere mittels geeignetem Schneidwerkzeug in das umgebende Muskelgewebe des Verletzten geschnitten wird. Bevorzugt wird eine Hülse (auch: Gewebeschutzhülse) in den derartig geschaffenen Zugang eingeführt, um das umliegende Muskelgewebe beim Eintreiben bzw. Einschrauben der Pins zu schützen. Nachdem die Pins in die jeweilig zu fixierenden Knochenpartien eingetrieben wurden, werden die Hülsen typischerweise entfernt und der externe Fixateur montiert, d. h. die Pins werden über Verbindungsvorrichtungen und einem Stabelement oder mehreren Stabelementen derart miteinander verbunden, dass zumindest die Stelle der Fraktur durch den externen Fixateur überbrückt ist. Dies erfolgt im Allgemeinen zur Herstellung der Transportfähigkeit des Verletzten, so dass dieser anschießend zur weiteren Behandlung in ein Krankenhaus oder Lazarett eingeliefert werden kann.

Für eine weitere Beschreibung der Erfindung wird auf die in den Zeichnungsfiguren gezeigten Ausführungsbeispiele verwiesen. Es zeigen in einer schematischen Darstellung:
- Fig. 1: ein mögliches Ausführungsbeispiel einer Verbindungsvorrichtung eines externen Fixateurs in einer Explosionsdarstellung;
- Fig. 2: die Verbindungsvorrichtung der Fig. 1 in einer möglichen räumlichen Konfiguration von der Seite;
- Fig. 3: die Verbindungsvorrichtung der Fig. 1 in einer weiteren möglichen räumlichen Konfiguration von der Seite;
- Fig. 4: die Verbindungsvorrichtung der Fig. 1 in einer weiteren möglichen räumlichen Konfiguration von der Seite;
- Fig. 5: die Verbindungsvorrichtung der Fig. 1 in einer möglichen räumlichen Konfiguration von der Seite;

- Fig. 6: die Verbindungsvorrichtung der Fig. 1 in einer weiteren möglichen räumlichen Konfiguration von der Seite;
- Fig. 7: die Verbindungsvorrichtung der Fig. 1 in einer weiteren möglichen räumlichen Konfiguration von der Seite;
- Fig. 8: eine Klemmbacke der Verbindungsvorrichtung der Fig. 1 im Detail in einer perspektivischen Ansicht;
- Fig. 9: die Klemmbacke der Fig. 8 in einer Draufsicht;
- Fig. 10: eine weitere Klemmbacke der Verbindungsvorrichtung der Fig. 1 im Detail in einer perspektivischen Ansicht;
- Fig. 11: die Klemmbacke der Fig. 10 in einer Draufsicht;
- Fig. 12: die Klemmbacke der Fig. 10 in einer Seitenansicht;
- Fig. 13: Details einer weiteren Klemmbacke der Verbindungsvorrichtung der Fig. 1 in einer Schnittdarstellung;
- Fig. 14: die Klemmbacke der Fig. 13 in einer perspektivischen Darstellung;
- Fig. 15: eine Gelenkkomponente der Verbindungsvorrichtung der Fig. 1 in einer perspektivischen Ansicht;
- Fig. 16: ein als Blattfeder ausgeführtes Federmittel der Verbindungsvorrichtung der Fig. 1 in einer perspektivischen Ansicht;
- Fig. 17: ein als Wellenfeder ausgeführtes Federmittel der Verbindungsvorrichtung der Fig. 1 in einer perspektivischen Ansicht;

- Fig. 18: der externe Fixateur in einer möglichen, beispielhaften Konfiguration zur Fixierung einer Fraktur des Oberschenkels;
- Fig. 19: zwei Stabelemente des externen Fixateurs, die mittels einer Verbindungsvorrichtung gemäß Fig. 1 miteinander verbunden sind, in einer perspektivischen Darstellung;
- Fig. 20: eine Einrichtung für die ambulante Notfallversorgung mit einer motorisch angetriebenen Befestigungsvorrichtung zum Eintreiben von Pins in das Knochengewebe eines Patienten.

Einander entsprechende Teile sind in allen Figuren mit den gleichen Bezugszeichen versehen.

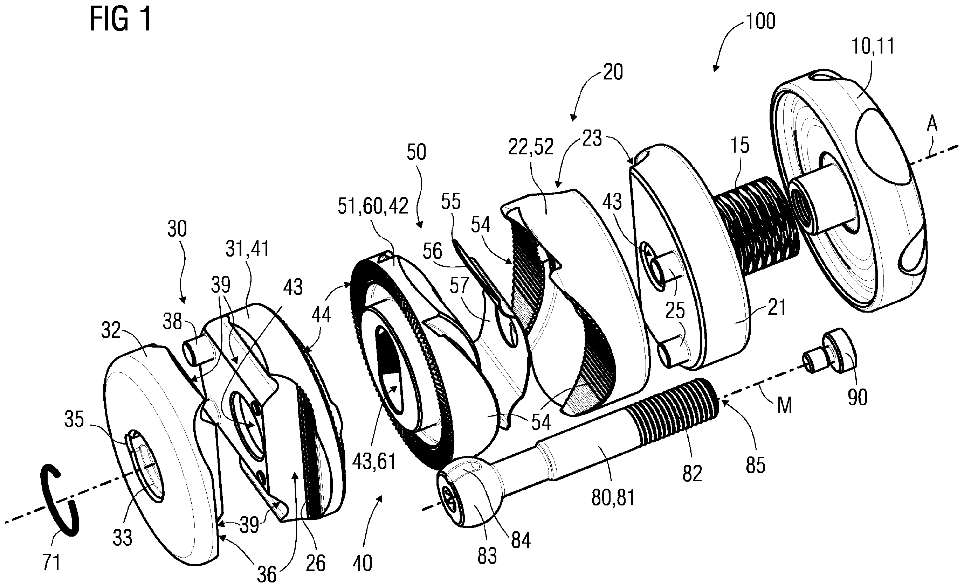

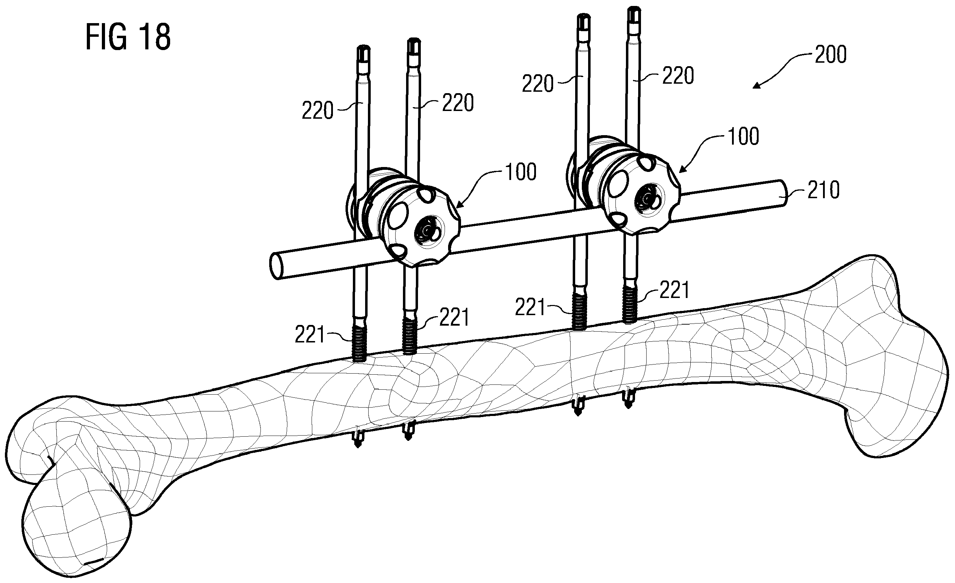

Figuren 1 bis 7 zeigen eine Verbindungsvorrichtung 100 eines externen Fixateurs 200, der in einer beispielhaften Konfiguration in Figur 18 illustriert ist.

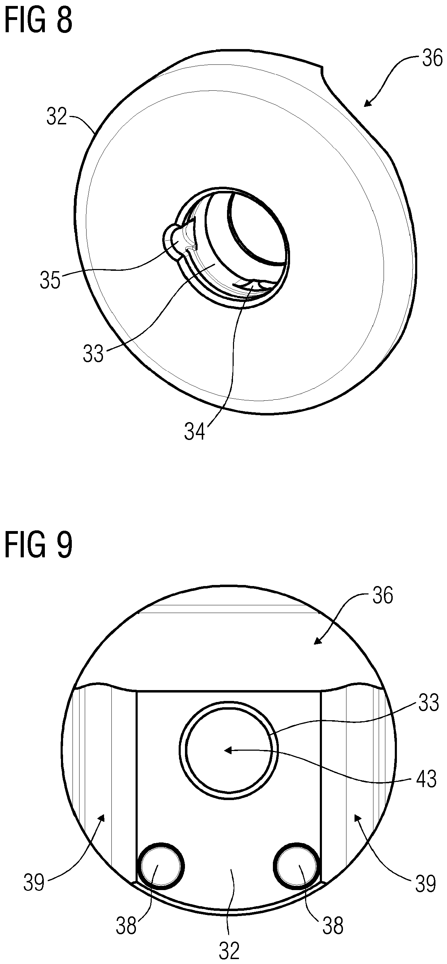

Figuren 8 bis 17 zeigen Details verschiedener Komponenten der Verbindungsvorrichtung 100.

Der externe Fixateur 200 umfasst, wie beispielsweise in Figur 18 gezeigt, neben den Verbindungsvorrichtungen 100 zumindest ein stangenförmiges Stabelement 210 und mehrere Pins 220, die als Gewindestangen dazu ausgebildet sind, im menschlichen oder tierischen Knochengewebe verankert zu werden und hierfür jeweils endseitig Bohrgewinden 211 aufweisen.

Die Verbindungsvorrichtung 100 ist dazu ausgebildet, Pins 220 und/oder Stabelemente 210 miteinander kraftschlüssig, optional unter zusätzlicher Zuhilfenahme eines Formschlusses, zu verbinden. Zu diesem Zweck weist die Verbindungsvorrichtung 100 erste und zweite Klemmenanordnungen 20, 30 mit jeweils gegenüberliegend angeordneten ersten und zweiten Klemmbacken 21, 22, 31, 32 auf, zwischen denen Pins 220 und/oder Stabelemente 210 durch Vermittlung einer in axialer Richtung A wirkenden Kraft verklemmt werden können.

Wie insbesondere in Figur 1 dargestellt, weist die Verbindungsvorrichtung 10 einen axialen Aufbau mit mehreren in etwa scheibenförmig ausgebildeten Komponenten auf, die zumindest in einer Grundstellung einander stirnseitig gegenüberliegend angeordnet sind. Insbesondere die vorstehend bereits genannten ersten und zweiten Klemmbacken 21, 22, 31, 32 der ersten und zweiten Klemmenanordnung 20, 30 und eine zwischen der zweiten Klemmbacke 22 der ersten Klemmenanordnung 20 und der ersten Klemmenbacke 31 der zweiten Klemmenanordnung 30 liegende Gelenckomponente 60 sind in etwa scheibenförmig ausgebildet und zueinander stirnseitig anordbar bzw. angeordnet. Ein Zugmittel 80 ist zentral durch die ersten und zweiten Klemmbacken 21, 22, 31, 32 der ersten und zweiten Klemmenanordnungen 20, 30 und der Gelenkkomponente 60 hindurchgeführt. Das Zugmittel 80 ist einstückig bzw. einteilig und starr ausgeführt.

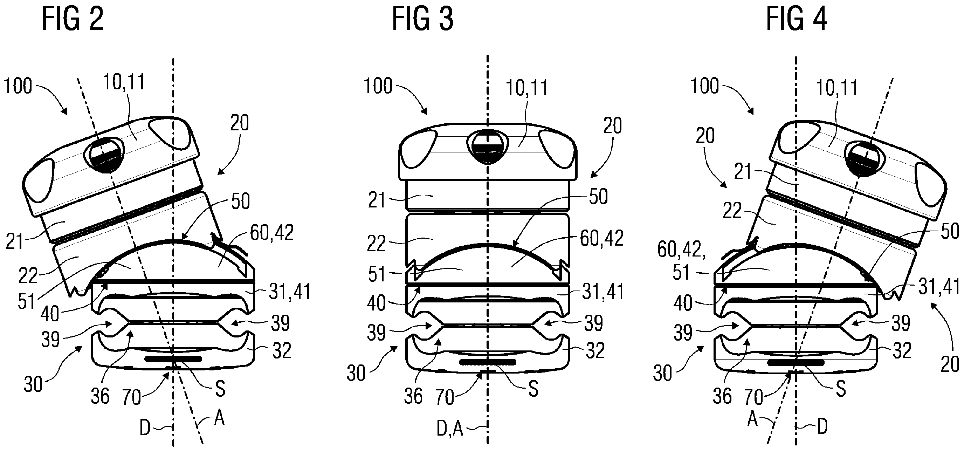

In dem dargestellten und nicht einschränkend auszulegenden Ausführungsbeispiel ist das Zugmittel als Schraube 81 mit einem Außengewinde 82 und einem in etwa kugelförmig ausgebildeten Kopfende 83 ausgeführt. Im Kopfende 83 sind zwei diametral gegenüberliegende Nuten 84 eingebracht. Das Kopfende 83 der Schraube 82 ist in einer Gelenkpfanne 33, welche in einem zentralen Bereich der zweiten Klemmbacke 32 der zweiten Klemmenanordnung 30 eingebracht ist, beweglich geführt. Das zumindest bereichsweise kugelförmig ausgebildete Kopfende 83 der Schraube 81 bildet mit der Gelenkpfanne 33 eine Gelenk 70, insbesondere nach Art eines Kugelgelenks, welches Schwenkbewegungen der Schraube 81 relativ zur zweiten Klemmbacke 32 der zweiten Klemmenanordnung 30 ermöglicht. Die Gelenkpfanne 33 weist zwei diametral gegenüberliegende Vorsprünge 34 oder Nasen auf, die im Detail in Figur 8 dargestellt sind. Die Vorsprünge 34 greifen im endmontierten Zustand der Verbindungsvorrichtung 100 in die Nuten 84 ein und unterbinden so eine Rotation der Schraube 81 um ihre Mittellängsachse M oder schränken diese zumindest ein. Die Ausrichtung der Mittellängsachse M entspricht im endmontierten Zustand der Verbindungsvorrichtung 100 der axialen Richtung A (vgl. insbesondere Figuren 2 bis 7). Im Bereich der Gelenkpfanne 33 ist eine weitere Nut 35 eingebracht (vgl. insbesondere Figur 1 oder 8), die zur Aufnahme eines Sicherungsrings 71 vorgesehen ist. Der Sicherungsring 71 ist dazu ausgebildet, ein Verschieben der Schraube 81 in axialer Richtung A, insbesondere bei der Montage der Verbindungsvorrichtung 100, zu unterbinden.

An dem Ende der Schraube 81, das dem Kopfende 83 gegenüberliegend angeordnet ist, ist ein Innengewinde 85 eingebracht, in das eine Sicherungsschaube 90 eingeschraubt ist. Die Sicherungsschraube 90 dient im endmontierten Zustand der Verbindungsvorrichtung 100 zur Sicherung eines Stellmittels 10, welches in dem dargestellten und nicht einschränkend auszulegenden Ausführungsbeispiel in der Art eines Handrads 11 ausgeführt ist. Das Handrad 11 ist auf dem Außengewinde 82 der Schraube 81 aufgeschraubt. Die Sicherungsschraube 90 limitiert den axialen Bewegungsspielraum des Handrads 11, insbesondere derart, dass das Handrad 11 im endmontierten Zustand nicht durch eine einfache Drehbewegung von dem Außengewinde 82 abgeschraubt werden kann.

Das Stellmittel 10 dient zur Vermittlung einer in axialer Richtung A wirkenden Zugbelastung bzw. Zugkraft. Durch Ausüben der Zugkraft in axialer Richtung A sind zwischen den Klemmbacken 21, 22, 31, 32 angeordnete Stabelemente 220 und/oder Pins 210 zumindest kraft- bzw. reibschlüssig fixierbar. Die ersten und zweiten Klemmbacken 21, 22 der ersten Klemmenanordnung 20 weisen gegenüberliegende und parallel zueinander verlaufende erste Aufnahmenuten 23 auf. Die ersten und zweiten Klemmbacken 31, 32 der zweiten Klemmenanordnung 30 weisen ebenfalls gegenüberliegende und parallel zueinander verlaufende erste Aufnahmenuten 36 auf. Die ersten Aufnahmenuten 23, 36 dienen zur Aufnahme von Stabelementen 220 und sind entsprechend dimensioniert. Durch Ausüben der axialen Zugbelastung können eingelegte Stabelementen 220 im Bereich der ersten Aufnahmenuten 23, 36 fixiert werden.

Die ersten und zweiten Klemmbacken 21, 22, 31, 32 der ersten und zweiten Klemmenanordnungen 20, 30 sind mit ineinander eingreifenden Strukturen versehen, die dazu ausgebildet sind, eine Rotation der ersten Klemmbacke 21 relativ zur zweiten Klemmbacke 22 der ersten Klemmenanordnung 20 bzw. eine Rotation der ersten Klemmbacke 31 relativ zur zweiten Klemmbacke 32 der zweiten Klemmenanordnung 30 zu unterbinden.

In dem dargestellten und nicht einschränkend auszulegenden Ausführungsbeispiel sind diese ineinander eingreifende Strukturen als sacklochartige Vertiefungen 24, 37 und Stifte 25, 38 ausgeführt.

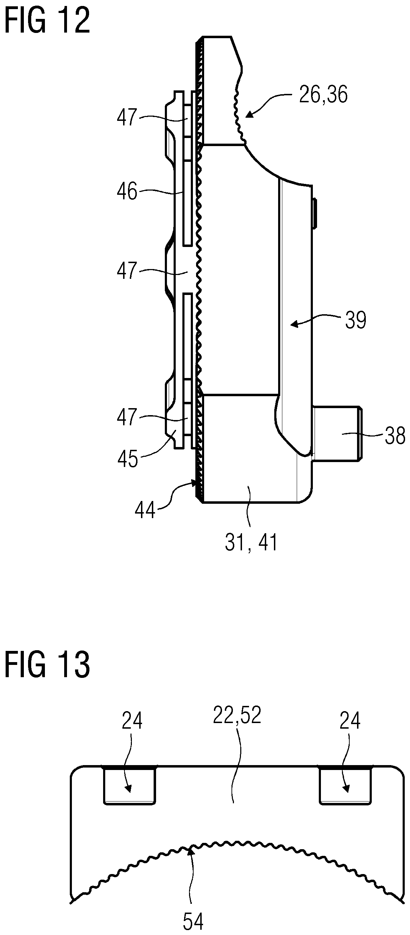

Figur 13 zeigt in einer Schnittdarstellung weitere Details der zweiten Klemmbacke 22 der ersten Klemmenanordnung 20. Die sacklochartigen Vertiefungen 24 sind in die Seite der zweiten Klemmbacke 22 eingebracht, die der ersten Klemmbacke 21 der ersten Klemmenanordnung 20 gegenüberliegend angeordnet ist. Die Stifte 25 stehen, wie insbesondere in Figur 1 gezeigt, stirnseitig der ersten Klemmbacke 21 hervor und greifen im endmontierten Zustand (vgl. insbesondere Figuren 2 bis 7) in die sacklochartigen Vertiefungen 24 ein.

Entsprechend sind sacklochartige Vertiefungen 38, wie insbesondere in Figur 9 dargestellt, in die zweite Klemmbacke 32 der zweiten Klemmenanordnung 30 eingebracht. Die Stifte 38 stehen stirnseitig der ersten Klemmbacke 31 der zweiten Klemmanordnung 30 hervor und greifen im endmontierten Zustand (vgl. beispielsweise Figuren 2 bis 7) in die sacklochartigen Vertiefungen 37 ein.

In anderen Ausgestaltungen sind die ineinander eingreifenden Strukturen invertiert ausgebildet, d.h. die Stifte 25, 38 können an den zweiten Klemmbacken 22, 32 und die sacklochartigen Vertiefungen 24, 37 an den ersten Klemmbacken 21, 31 der ersten und/oder zweiten Klemmenanordnung 20, 30 vorgesehen sein.

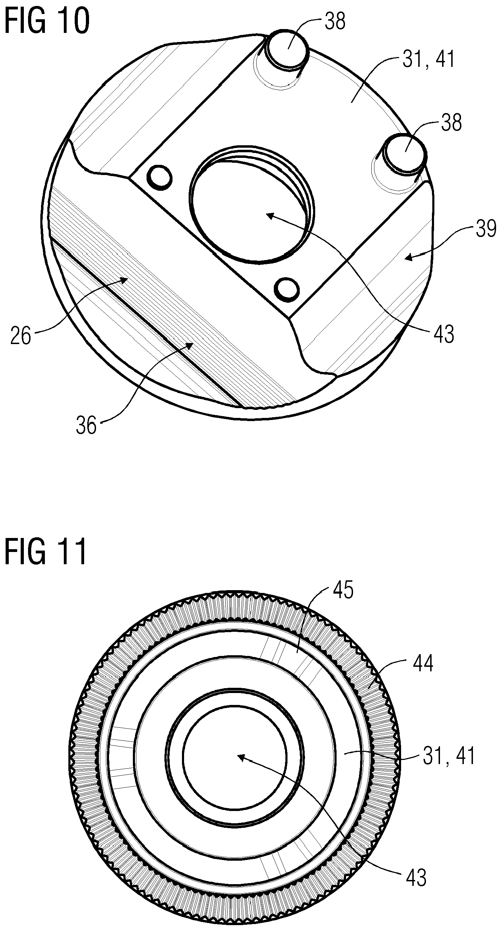

In dem dargestellten und nicht einschränkend auszulegenden Ausführungsbeispiel sind die ersten Aufnahmenuten 23, 36 abschnittsweise mit dritten Oberflächenstrukturierungen 26 versehen (vgl. beispielsweise Figur 1 oder Figur 10), die dazu ausgebildet sind, in komplementär ausgebildete Oberflächenstrukturierungen eines Stabelements 210 formschlüssig einzugreifen. Die dritte Oberflächenstrukturierung 26 umfasst mehrere parallel zueinander verlaufende Rillen und Rippen, die sich längs der Aufnahmenuten 23, 36 erstrecken. Die Fixierung der Stabelemente 210 erfolgt somit unter zusätzlicher Zuhilfenahme eines Formschlusses.

Die zweite Klemmenanordnung 30 weist in dem dargestellten und nicht einschränkend auszulegenden Ausführungsbeispiel zusätzlich zu den ersten Aufnahmenuten 36 zweite Aufnahmenuten 39 auf (vgl. insbesondere Figur 9 oder 10), die zur Aufnahme von Pins 210 ausgelegt sind und hierfür beispielsweise etwas kleiner dimensioniert sind. Durch Ausüben der axialen Zugbelastung sind die eingelegten Pins 210 im Bereich der zweiten Aufnahmenuten 31 fixierbar.

In dem dargestellten und nicht einschränkend auszulegenden Ausführungsbeispiel sind zwei zweite Aufnahmenuten 39 in die erste Klemmbacke 31 der zweiten Klemmenanordnung 30 und zwei zweite Aufnahmenuten 39 in die zweite Klemmbacke 32 der zweiten Klemmenanordnung 30 eingebracht. Die in die erste Klemmbacke 31 eingebrachten, zweiten Aufnahmenuten 39 sind den Aufnahmenuten 39 gegenüberliegend angeordnet, die in der zweiten Klemmbacke 32 eingebracht sind. Die zweiten Aufnahmenuten 39 verlaufen parallel zueinander, so dass in der zweiten Klemmenanordnung 30 zwei Pins 210 in paralleler Ausrichtung zueinander kraft- und reibschlüssig fixiert werden können. Die zweite Aufnahmenuten 39 sind im dargestellten Ausführungsbeispiel senkrecht zu den ersten Aufnahmenuten 36 der zweiten Klemmenanordnung 30 orientiert, Abweichungen hiervon sind jedoch möglich und in alternativen Ausgestaltungen vorgesehen.

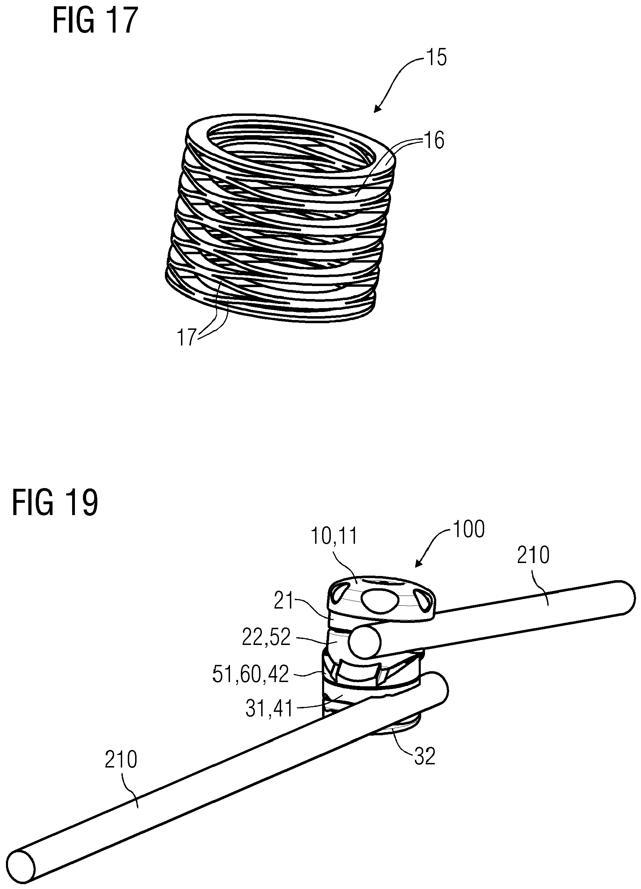

Zwischen dem Stellmittel 10 und der ersten Klemmbacke 21 der ersten Klemmenanordnung 20 ist eine Feder 15 angeordnet, die beispielsweise, wie insbesondere in Figur 1 dargestellt, als Schrauben- oder Druckfeder, oder, wie insbesondere in Figur 17 gezeigt, als Wellenfeder ausgeführt ist. Die Feder 15 stellt eine gewisse Beweglichkeit der ersten und zweiten Klemmbacken 21, 22, 31, 32 der ersten und zweiten Klemmenanordnungen 20, 30 sicher, wenn das Zugmittel nicht oder nur geringfügig unter Zugbelastung steht. Dies ermöglicht eine gewisse Beweglichkeit der Klemmbacken 21, 22, 31, 33 bei geringer axialer Belastung derart, dass beispielsweise die Pins 220 und/oder die Stabelemente 210 einfach in lateraler Richtung in die entsprechenden zur Aufnahme vorgesehenen ersten und/oder zweiten Aufnahmenuten 23, 36, 39 eingeclipst werden können. Die von den ersten und/oder zweiten Aufnahmenuten 23, 36, 39 gebildeten Aufnahmen bilden somit eine Art Snap-in Verbindung, die durch Vermittlung der längs des Zugmittels 80 wirkenden Zugbelastung arretiert werden kann.

Die in Figur 17 gezeigte Ausführungsvariante der Feder 15 weist eine im wesentlichen zylindrische Gestalt auf und ist in vorteilhafter Weise dazu ausgelegt, Querbelastungen, die in einer von der axialen Richtung A abweichenden Richtung wirken, aufzunehmen. Die als Wellenfeder ausgebildete Feder 15 umfasst mehrere Ringelemente 16, die über zumindest abschnittsweise flexibel ausgebildete Stege 17 miteinander verbunden sind.

Die erste Klemmenanordnung 20 und die zweite Klemmenanordnung 30 sind über ein Drehgelenk 40 zueinander um eine Drehachse D drehbar und über ein Schwenkgelenk 50 zueinander um eine Schwenkachse S schwenkbar gelagert. In dem in den Zeichnungsfiguren dargestellten und nicht einschränkend auszulegenden Ausführungsbeispiel sind die erste Klemmenanordnung 20 und zweite Klemmenanordnung 30 relativ zueinander um Drehachse D über den vollen Winkelbereich von 360° verstellbar. Bezüglich der Schwenkachse S sind die erste Klemmenanordnung 20 und zweite Klemmenanordnung 30 relativ zueinander über einen eingeschränkten Winkelbereich von etwa 40° verstellbar (vgl. insbesondere Figuren 2 und 4).

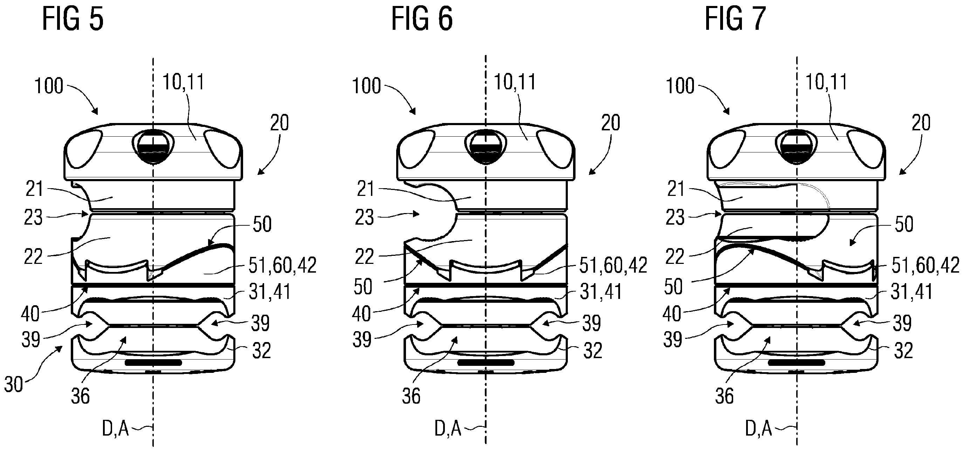

Die erste Klemmenanordnung 20 und die zweite Klemmenanordnung 30 sind durch Vermittlung der längs des Zugmittels 83 in axialer Richtung A wirkenden Zugbelastung bezüglich der Drehachse D und der Schwenkachse S in unterschiedlichen Winkelstellungen arretierbar. Figuren 2 bis 4 zeigen exemplarisch drei unterschiedliche Winkelstellungen, in denen die erste Klemmenanordnung 20 relativ zur zweite Klemmenanordnung 30 in unterschiedlichen Winkelstellungen um die Schwenkachse S arretiert werden kann. Entsprechend zeigen Figuren 5 bis 7 exemplarisch drei unterschiedliche Winkelstellungen, in denen die erste Klemmenanordnung 20 relativ zur zweite Klemmenanordnung 30 in unterschiedlichen Winkelstellungen um die Drehachse D arretiert werden kann.

Das Drehgelenk 40 umfasst, wie insbesondere in Figur 1 dargestellt, zwei in axialer Richtung A einander stirnseitig gegenüberliegend angeordnete Drehgelenkkomponenten 41, 42, die zueinander und um Drehachse D drehbar gelagert sind. Das Zugmittel 80 ist zentral durch das Drehgelenk 40 hindurchgeführt. Hierzu weisen die Drehgelenkkomponenten 41, 42 zentrale Bohrungen 43 auf, in denen das Zugmittel 80 eingesetzt ist. Die stirnseitig gegenüberliegenden Seiten der Drehgelenkkomponenten 41, 42 sind ferner mit ersten Oberflächenstrukturierungen 44 (vgl. insbesondere Figuren 1 und 11) versehen, die zueinander komplementär ausgebildet sind und einen Formschluss zwischen den Drehgelenkkomponenten 41, 42 bereitstellen. Die ersten Oberflächenstrukturierungen 44 weisen im Wesentlichen gezahnte oder geriffelte Strukturen auf. In dem dargestellten und nicht einschränkend auszulegenden Ausführungsbeispiel sind die ersten Oberflächenstrukturierungen 44 als axial vorstehende, umlaufende Zahnstrukturen, insbesondere mit Radialsymmetrie, ausgeführt, die einander gegenüberliegend angeordnet sind und formschlüssig ineinander eingreifen, wenn die Drehgelenkkomponenten 41, 42 zueinander bezüglich der Drehachse D in unterschiedlichen Winkelstellungen positioniert und durch Vermittlung eine axial wirkenden Kraft arretiert werden.

Zwischen den Drehgelenkkomponenten 41, 42 ist ein erstes Federmittel 45 angeordnet, welches dazu ausgebildet ist, die Drehgelenkkomponenten 41, 42 entgegen der mittels des Zugmittels 80 vermittelbaren Zugbelastung federnd vorzuspannen. Das erste Federmittel 45 ist im in Figur 12 dargestellten und nicht einschränkend auszulegenden Ausführungsbeispiel einstückig mit der Drehgelenkkomponente 41 ausgebildet und steht dieser in axialer Richtung A hervor. Das erste Federmittel 45 ist insbesondere zur Aufnahme von Querbelastung ausgelegt und zumindest abschnittsweise deformierbar ausgebildet. Hierzu weist das erste Federmittel 45 ein Ringelement 46 auf, welches über Stege 47 mit der Drehgelenkkomponente 41 verbunden ist.

In hierzu alternativen Ausgestaltungen ist das erste Federmittel 45 als separates Bauteil realisiert. Beispielsweise ist das erste Federmittel 45 als Wellenfeder ausgeführt und weist zwei konzentrisch zueinander und um die axiale Richtung A angeordnete Ringe oder Ringelemente auf, die über axial verlaufende Stege miteinander verbunden sind. Das erste Federmittel 45 kann beispielsweise aus Titan bestehen.

Die Drehgelenkkomponente 41 und die erste Klemmbacke 31 der zweiten Klemmenanordnung 30 sind in dem dargestellten und nicht einschränkend auszulegenden Ausführungsbeispiel als ein einziges Bauteil ausgebildet. In alternativen Ausführungsbeispielen sind die Drehgelenkkomponente 41 und die erste Klemmbacke 31 beispielsweise als separate Bauteile realisiert.

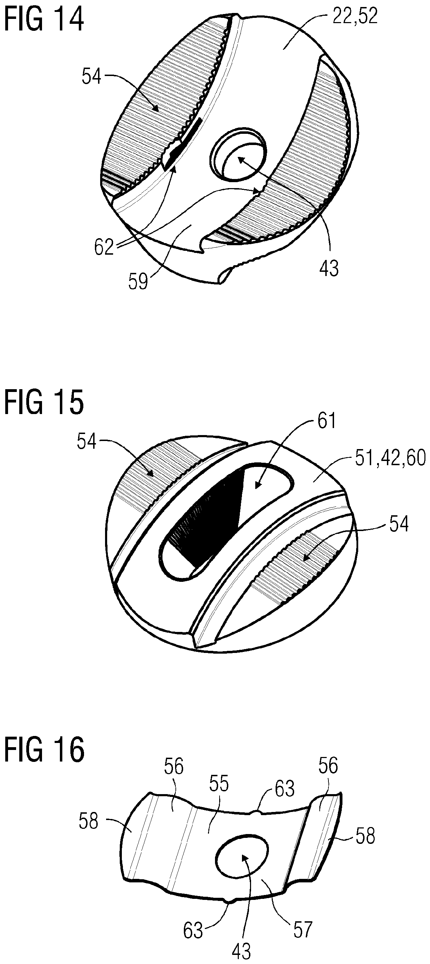

Die Drehgelenkkomponente 42 bildet in dem dargestellten und nicht einschränkend auszulegenden Ausführungsbeispiel zudem eine Schwenkgelenkkomponente 51 des Schwenkgelenks 50 und kann daher zusammenfassend auch als Gelenkkomponente 60 bezeichnet werden, die in einer perspektivischen Darstellung in Figur 15 gezeigt ist. In alternativen Ausführungsbeispielen sind die Drehgelenkkomponente 42 und die Schwenkgelenkkomponente 51 beispielsweise als separate Bauteile realisiert.

Das Schwenkgelenk 50 umfasst zwei in axialer Richtung A einander stirnseitig gegenüberliegend angeordnete Schwenkgelenkkomponenten 51, 52, die zueinander und um Schwenkachse S schwenkbar gelagert sind. Das Zugmittel 80 ist zentral durch das Schwenkgelenk 40 hindurchgeführt. Die Bohrung 43 in der Schwenkgelenkkomponente 51 ist als Langloch 61 ausgebildet (vgl. insbesondere Figur 15), das eine Art Kulisse bildet, entlang der das zentral geführte Zugmittel 80 geführt wird, wenn die ersten und zweiten Klemmenanordnungen 20, 30 zueinander bezüglich der Schwenkachse S verstellt werden. Die Schwenkgelenkkomponenten 51, 52 und die Klemmbacken 21, 22, 31, 32 sind ebenfalls mit zentralen Bohrungen 43 versehen, durch die das Zugmittel 80 hindurchgeführt ist. Die stirnseitig gegenüberliegenden Seiten der Schwenkgelenkkomponenten 51, 52 sind bezüglich der axialen Richtung A zumindest bereichsweise derart konvex bzw. konkav gekrümmt, dass eine zumindest kraft- oder reibschlüssige Arretierung der Schwenkgelenkkomponenten 51, 52 zueinander bezüglich der senkrecht zur Drehachse D verlaufenden Schwenkachse S ermöglicht ist. Der Krümmungsradius der konvex oder konkav gekrümmten Abschnitte ist insbesondere konstant.

Die stirnseitig gegenüberliegenden Seiten der Schwenkgelenkkomponenten 51, 52 sind in dem in den Zeichnungsfiguren dargestellten und nicht einschränkend auszulegenden Ausführungsbeispiel ferner mit zweiten Oberflächenstrukturierungen 54 versehen (vgl. insbesondere Figuren 14 und 15), die zueinander komplementär ausgebildet sind und einen Formschluss zwischen Drehgelenkkomponenten 51, 52 bereitstellen. Die zweiten Oberflächenstrukturierungen 54 weisen im Wesentlichen gezahnte oder geriffelte Strukturen auf. In dem dargestellten und nicht einschränkend auszulegenden Ausführungsbeispiel weisen die zweiten Oberflächenstrukturierungen 54 mehrere komplementär zueinander ausgebildete Rippen und Rillen auf, die parallel zueinander und parallel zur Schwenkachse S angeordnet sind. Die zweiten Oberflächenstrukturierungen 54 greifen formschlüssig ineinander ein, wenn die Schwenkgelenkkomponenten 51, 52 zueinander bezüglich der Schwenkachse S in unterschiedlichen Winkelstellungen positioniert werden.

Zwischen den Schwenkgelenkkomponenten 51, 52 ist ein zweites Federmittel 55 angeordnet, welches dazu ausgebildet ist, die Schwenkgelenkkomponenten 51, 52 entgegen der mittels des Zugmittels 80 vermittelbaren Zugbelastung federnd vorzuspannen. Das zweite Federmittel 55 ist im dargestellten und nicht einschränkend auszulegenden Ausführungsbeispiel nach Art einer Blattfeder ausgeführt und weist, wie insbesondere in Figur 16 gezeigt, mehrere Abschnitte 56, 57, 58 auf, die bezüglich der axialen Richtung A unterschiedlich gekrümmt sind. Die radial außenliegenden Abschnitte 56 sind beispielsweise entgegengesetzt zum zentralen Abschnitt 57 und zu endseitigen Abschnitten 58 gekrümmt, so dass die durch das zweite Federmittel 55 vermittelte Federkraft die Schwenkgelenkkomponenten 51, 52 in axialer Richtung A auseinandertreibt.

Das zweite Federmittel 55 ist im endmontierten Zustand in einem zentralen Bereich zwischen den Schwenkgelenkkomponenten 51, 52 angeordnet. Im zentralen Bereich ist die Schwenkgelenkkomponente 51, wie insbesondere in Figur 15 dargestellt, beispielsweise erhöht ausgebildet. Entsprechend weist die Schwenkgelenkkomponente 52 im zentralen Bereich beispielsweise eine Vertiefung 59 auf. Am Rand der Vertiefung 59 sind Aufnahmen 62 angeordnet, die zur Aufnahme von seitlichen Vorsprüngen 63 des zweiten Federmittels 55 dienen, so dass dieses im zentralen Bereich zwischen den Schwenkgelenkkomponenten 51, 52 fixiert werden kann.

Figur 18 zeigt den externen Fixateur 200 in einer möglichen Konfiguration zur Stabilisierung eines Oberschenkelbruchs. Insgesamt vier Pins 221 sind in das Knochengewebe des Oberschenkels eingetrieben worden. Jeweils zwei Pins 220 sind an je einer Verbindungsvorrichtung 100 befestigt. Die Pins 220 sind über ein Stabelement 210 miteinander verbunden. Das Stabelement 210 weist vorzugsweise eine vierte Oberflächenstrukturierung 212 auf, die komplementär zur dritten Oberflächenstrukturierung 26 ist, so dass das Stabelement 210 in den Aufnahmenuten 23, 36 kraft- und formschlüssig befestigt sind.

Figur 19 zeigt eine Konfiguration, in der zwei Stabelemente 210 mittels einer Verbindungsvorrichtung 100 miteinander verbunden wurden. Die Stabelemente 210 sind in den ersten Aufnahmenuten 23, 39 der ersten und zweiten Klemmenanordnungen 20, 30 kraft- und formschlüssig gehalten. In der exemplarisch dargestellten Konfiguration sind die ersten und zweiten Klemmenanordnungen 20, 30 zueinander sowohl bezüglich der Drehachse D als auch der Schwenkachse S verstellt. Die zweiten Aufnahmenuten 39 zur Aufnahmen von Pins 220 bleiben typischerweise ungenutzt, wenn an der Verbindungsvorrichtung 100 zwei Stabelemente 210 befestigt werden.

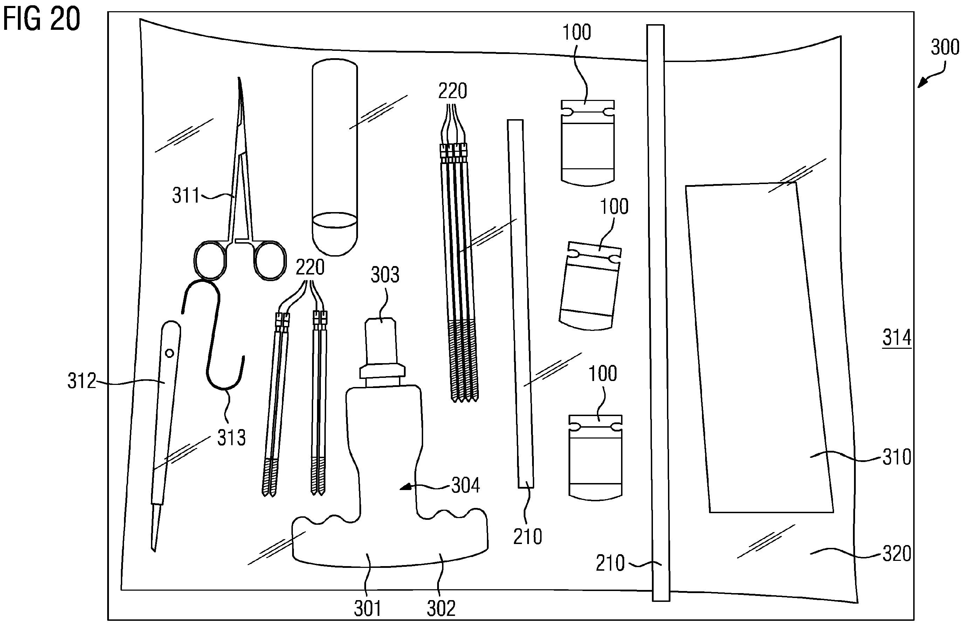

Figur 20 zeigt eine Einrichtung 300 für die ambulante Notfallversorgung, die die Komponenten des externen Fixateurs 200, also insbesondere die bereits beschriebenen Verbindungsvorrichtungen 100, Stabelemente 210 und Pins 220, insbesondere Pins 220 mit unterschiedlichen axialen Längen und Durchmessern, und weitere Komponenten aufweist, die zur Durchführung der medizinischen Notfallmaßnahme notwendig oder hilfreich sind. Die Einrichtung 300 umfasst hierzu insbesondere eine motorisch angetriebene Befestigungsvorrichtung 301, die zum Einschrauben der Pins 220 in Knochengewebe ausgebildet ist.

Die Befestigungsvorrichtung 301 weist ein T-förmiges Griffstück 302 auf und ist insbesondere dazu ausgelegt, von einem Benutzer mit einer einzelnen Hand bedient zu werden. Die Befestigungsvorrichtung 301 ist als elektrischer Schraube ausgeführt und weist eine Aufnahme 303 in der Art eines Spannfutterals auf, welches dazu ausgebildet ist, die in das Knochenmaterial der zu fixierenden Knochenpartien einzutreibenden Pins 220 endseitig am Griffstück 302 kraftschlüssig zu fixieren. Als Energiespeicher 304 der Befestigungsvorrichtung 301 ist eine Einwegbatterie oder eine wiederaufladbare Batterie vorgesehen, die innerhalb des Griffstücks 302 angeordnet ist.

Als zusätzliche Komponenten bzw. medizinisches Zubehör umfasst die Einrichtung 300 beispielsweise eine Wundauflage 310, eine Klemme 311, ein Skalpell 312 und einen S-förmigen Haken 313, der insbesondere zur Befestigung von Infusionsbeuteln oder dergleichen dienen kann. Ferner ist ein Tuch bzw. eine textile Unterlage 314 enthalten, auf der die medizinische Notfallmaßnahme durchgeführt werden kann, um das Risiko einer Verunreinigung der Wunde zumindest zu reduzieren.

Es versteht sich, dass diese Aufzählung nicht abschließend ist und gegebenenfalls weitere oder andere Komponenten zur Durchführung der Notfallmaßnahme im Rahmen der Einrichtung 300 zur ambulanten Notfallversorgung beinhaltet sein kann.

Zumindest einige der Komponenten der medizinischen Einrichtung 300, insbesondere solche Komponenten, die bei der Durchführung der Notfallmaßnahme in direkten Kontakt mit dem Gewebe des Verletzten kommt, wie beispielsweise die Pins 220, die Wundauflage 310 oder die Klemme 311, sind vorzugsweise sterilisiert in einer Kunststoffhülle 320 eingeschweißt.

Die ambulante Notfallversorgung eines Bruches mit Hilfe des externen Fixateurs 200 erfolgt vorzugsweise auf der textilen Unterlage 314, welche Bestandteil der Einrichtung 300 ist. Zunächst müssen die Pins 220 in die zu fixierenden Knochenfragmente eingetrieben werden. Hierzu ist es gegebenenfalls notwendig, einen Zugang durch umliegendes Muskelgewebe schaffen, was beispielsweise mit Hilfe des Skalpells 312 erfolgen kann. Die Klemme 311 kann insbesondere dazu dienen, den Zugang offen zu halten, um eine Gewebeschutzhülse einzusetzen. Anschließend wird ein geeigneter Pin 220 in die Gewebeschutzhülse eingeführt, und der Pin 220 unter Verwendung der Befestigungsvorrichtung 301 eingetrieben. Dabei wird die Befestigungsvorrichtung 301 von einem Benutzer typischerweise mit einer Hand bedient, so dass die andere Hand gegebenenfalls zum Fixieren des verletzten Körperteils verbleibt. Dieser Vorgang wird wiederholt, bis eine ausreichende Anzahl von Pins 220 in alle der zu fixierenden Knochenfragmente eingetrieben wurden. Anschließend werden die mit den Knochenpartien verbundenen Pins 220 außerhalb des Körpers des Patienten über eine geeignete Anzahl von Verbindungsvorrichtungen 100 und Stabelementen 210 miteinander verbunden, um eine Relativbewegung der Knochenfragmente zueinander weitestgehend zu unterbinden und die Transportfähigkeit des Patienten herzustellen.

Obwohl die Erfindung im Detail mit Bezug auf die bevorzugten Ausführungsbeispiele näher illustriert und beschrieben wurde, so ist die Erfindung nicht hierdurch eingeschränkt. Andere Variationen und Kombinationen können vom Fachmann hieraus abgeleitet werden, ohne vom wesentlichen Gedanken der Erfindung abzuweichen. Insbesondere sind beliebige Kombinationen von Merkmalen möglich, die mit Bezug auf verschiedene Ausführungsbeispiele und/oder Figuren beschrieben oder offenbart wurden.

Beispielsweise können die zweiten Aufnahmenuten 39 zur Befestigung von Pins 220 alternativ an der ersten Klemmenanordnung 20 vorgesehen sein oder sowohl die erste Klemmenanordnung 20 als auch die zweite Klemmenanordnung 30 mit zweiten Aufnahmenuten 39 zur Befestigung von Pins 220 versehen sein. Variationen hinsichtlich der Formgebung der ineinander eingreifenden Strukturen und/oder der Oberflächenstrukturierungen, insbesondere der ersten, zweiten und/oder dritten Oberflächenstrukturierungen sind möglich und vorgesehen.

### Bezugszeichen liste

- 10: Stellmittel
- 11: Handrad
- 15: Feder
- 16: Ringelement
- 17: Steg
- 20: Klemmenanordnung
- 21: Klemmbacke
- 22: Klemmbacke
- 23: Aufnahmenut
- 24: Vertiefung
- 25: Stift
- 26: Oberflächenstrukturierung
- 30: Klemmenanordnung
- 31: Klemmbacke
- 32: Klemmbacke
- 33: Gelenkpfanne
- 34: Vorsprung
- 35: Nut
- 36: Aufnahmenut
- 37: Vertiefung
- 38: Stift
- 39: Aufnahmenut
- 40: Drehgelenk
- 41: Drehgelenkkomponente
- 42: Drehgelenkkomponente
- 43: Bohrung
- 44: Oberflächenstrukturierung
- 45: Federmittel
- 46: Ringelement
- 47: Steg
- 50: Schwenkgelenk

- 51: Schwenkgelenkkomponente
- 52: Schwenkgelenkkomponente
- 54: Oberflächenstrukturierung
- 55: Federmittel
- 56: Abschnitt
- 57: Abschnitt
- 58: Abschnitt
- 59: Vertiefung
- 60: Gelenkkomponente
- 61: Langloch
- 62: Aufnahme
- 63: Vorsprung
- 70: Gelenk
- 71: Sicherungsring
- 80: Zugmittel
- 81: Schraube
- 82: Außengewinde
- 83: Kopfende
- 84: Nut
- 85: Innengewinde
- 90: Sicherungsschraube
- 100: Verbindungsvorrichtung
- 200: externer Fixateur
- 210: Stabelement
- 212: Oberflächenstrukturierung
- 220: Pin
- 221: Bohrgewinde
- 300: Einrichtung
- 301: Befestigungsvorrichtung
- 302: Griffstück
- 303: Aufnahme

- 304: Energiespeicher
- 310: Wundauflage
- 311: Klemme
- 312: Skalpell
- 313: Haken
- 314: Unterlage
- 320: Kunststoffhülle
- A: axiale Richtung
- M: Mittellängsachse
- D: Drehachse
- S: Schwenkachse

## Patentansprüche

1. Einrichtung (300) für die ambulante Notfallversorgung, umfassend zumindest die folgenden Komponenten:
- einen externen Fixateur (200) mit
- zumindest zwei, beispielsweise vier Pins (220), die zur Verankerung, insbesondere durch Einschrauben, in Knochengewebe ausgebildet sind,
- zumindest ein Stabelement (210) und
- zumindest eine Verbindungsvorrichtung (100), insbesondere zumindest zwei, beispielsweise drei Verbindungsvorrichtungen (100), die dazu ausgebildet sind, zumindest einen der Pins (220) mit dem zumindest einen Stabelement (210) mechanisch zu verbinden, **gekennzeichnet durch**
- eine motorisch angetriebene Befestigungsvorrichtung (301) zum Eintreiben der Pins (220) in das Knochengewebe, wobei die Befestigungsvorrichtung (301) zur einhändigen Bedienung durch einen Benutzer ausgebildet ist.

2. Einrichtung (300) nach Anspruch 1, wobei die Befestigungsvorrichtung (301) als elektrischer Schrauber mit T-förmigen Griffstück (302) ausgebildet ist.

3. Einrichtung (300) nach Anspruch 1 oder 2, wobei die Befestigungsvorrichtung (301) eine Aufnahme (303) zur kraftschlüssigen Befestigung eines der Pins (220) aufweist.

4. Einrichtung (300) nach einem der vorhergehenden Ansprüche, wobei die Befestigungseinrichtung (301) einen Energiespeicher (304), insbesondere eine Einwegbatterie oder eine wiederaufladbare Batterie, aufweist.

5. Einrichtung (300) nach einem der vorhergehenden Ansprüche, mit zumindest zwei Stabelementen (210), die optional unterschiedliche axiale Längen aufweisen, wobei die zumindest eine Verbindungsvorrichtung (100) dazu ausgebildet ist, die zumindest zwei Stabelemente (210) miteinander mechanisch zu verbinden.

6. Einrichtung (300) nach einem der vorhergehenden Ansprüche, wobei zumindest zwei der Pins (220) unterschiedliche axiale Längen und/oder Durchmesser aufweisen.

7. Einrichtung (300) nach einem der vorhergehenden Ansprüche, wobei zumindest einige der Komponenten der Einrichtung (300) in einer Kunststoffhülle (320) eingeschweißt sind.

8. Einrichtung (300) nach einem der vorhergehenden Ansprüche, wobei die in der Kunststoffhülle (320) eingeschweißten Komponenten sterilisiert oder sterilisierbar sind.

9. Einrichtung (300) nach einem der vorhergehenden Ansprüche, mit einer textilen Unterlage (314) zur Bereitstellung einer Eingriffsumgebung.

10. Einrichtung (300) nach einem der vorhergehenden Ansprüche, wobei die zumindest eine Verbindungsvorrichtung (100) dazu ausgebildet ist, die zumindest zwei Pins (220) mit zumindest einem der Stabelemente (210) mechanisch zu verbinden.

11. Einrichtung (300) nach Anspruch 10, wobei die zumindest eine Verbindungsvorrichtung (100) dazu ausgebildet ist, die zumindest zwei Pins (220) in paralleler Ausrichtung mit zumindest einem der Stabelemente (210) mechanisch zu verbinden.

12. Einrichtung (300) nach einem der vorhergehenden Ansprüche, wobei die mechanische Verbindung unter Vermittlung eines Formschlusses erfolgt, der durch einander komplementär ausgebildete Oberflächenstrukturierungen (26) vermittelt wird.
